# EUROPEAN PATENT APPLICATION

(11) **EP 4 151 619 A1**
(43) Date of publication of application: **22.03.2023**
(21) Application number: 21197670.9
(22) Date of filing: 20.09.2021
(51) Int. Cl.: C07C 209/84, C07C 263/10, C08G 69/00

(54) **METHOD FOR THE REMOVAL OF WATER FROM AND TRANSPORT OF ALIPHATIC DIAMINES**

(71) Applicant: Covestro Deutschland AG, 51373 Leverkusen (DE); Genomatica, Inc., San Diego CA 92121 (US)
(72) Inventor: MERKEL, Michael, 40223 Düsseldorf (DE); LEIMBRINK, Mathias, 44139 Dortmund (DE); STEFFENS, Friedhelm, 51373 Leverkusen (DE)
(74) Representative: Scholz, Volker

(57) **Abstract**

The invention relates to a method for the removal of water from and transport of at least one aliphatic diamine comprising the following steps:
a) providing a first composition including 60 to 98 wt.-% water and 2 to 40 wt.-% of the at least one aliphatic diamine with two -NH₂ groups per molecule, each of them bound to a primary or secondary carbon atom,
b) removing water at least partially from said first composition by distillation at a first production location in a distillation apparatus comprising at least two distillation columns operating at different head pressures, thus generating a second composition comprising the diamine and ≤ 55 wt.-% water, wherein the vapours emerging from the distillation column operating at the higher head pressure are used to evaporate the liquid in the bottoms and/or the feed of a distillation column operating at the lower head pressure at least partially,
c) transporting said second composition during a span of time t from the first production location to a second production location, wherein the span of time t including times for optional temporary storage is at least 6 h.

## Description

The invention relates to a method for the removal of water from and transport of at least one aliphatic diamine. The invention also relates to a method for the production of a polyamide, an aliphatic diisocyanate or a diamine-dicarboxylic acid salt and a second composition obtainable or produced by the method of the invention.

Aliphatic diamines are an important starting material for the production of polymers. They are used for example for the production of polyamides by polycondensing the diamine with dicarboxylic acids. Polyamide-6.6 (PA66), for example, is generated from hexane-1,6-diamine and adipic acid. Another important use is the phosgenation of the diamines to diisocyanates, which can then be converted in a further step into polyurethanes, polyureas or polyisocyanurates for example. Phosgenation places special demands on the purity of the aliphatic diamine.

Certain requirements to be met by the diamine are known, for example, for the production of hexamethylene diisocyanate from hexane-1,6-diamine. EP2060560B1 describes that isocyanates with particularly bright colours can be produced from amines whose PRI value, i.e. the content of polarographically reducible compounds, is less than 60 mol-ppm.

Since the supply of resources is decreasing, the chemical industry is paying ever greater attention to recycling plastics. One possible method here is chemical hydrolysis, by which the diamine building blocks can be recovered from the corresponding plastics, for example from polyurethanes or polyamides. These then first appear in the form of aqueous solutions.

For pentane-1,5-diamine, industrial production methods have become established for some years, in which the diamine is produced by biotechnological means, where first of all it is generated as a mixture with water, which is then processed into pure pentane-1,5-diamine. Biotechnological production processes are also known for hexane-1,6-diamine, and corresponding processes and microorganisms are disclosed in US20170369913A1 and WO2016209883A1 for example.

Both for recycling and for the biotechnological production of aliphatic diamines however, it is the case that the raw materials needed are frequently not available at the locations where derivatives of the diamines are to be manufactured later. Especially for isocyanates and polyamides, which are produced in large quantities, economic constraints mean that there are nowadays often only a small number of very large world-scale plants. This results in the need to transport the diamines over long distances.

Because of the large quantities to be transported and the fact there is often only a small amine content in the aqueous solutions produced, it is advisable first to remove water from the amines before they are transported to their destination, so that there is no need to transport large quantities of water. This is necessary not least because the transport process would otherwise have a negative impact on the environmental balance of the products and reduce the benefits obtained by using recycled materials or renewable resources. The removal of water is generally one of the first steps performed in the process of treating the raw diamine products formed. Distillation in a multiple column is known to be a suitable method for removing water, so that water is separated over the head and an amine from which most of the water has been removed is left at the bottom of the column.

Especially for diamines, which occur dissolved in a large amount of water, it is desirable to keep the energy consumption to a minimum by removing water.

One possible way to achieve this is described in EP2684867A1 for example. There, a method for the production of pentane-1,5-diamine is disclosed, in which the diamine is extracted in a liquid phase extraction process from the aqueous phase that has optionally been concentrated by distillation beforehand. Non-halogen aliphatic solvents, preferably straight chain alcohols with 4 to 7 carbon atoms, are recommended as extractants. These extractants must subsequently be separated from the product again by distillation. The fact that the heat of evaporation of the extractants is usually lower than that of water makes it possible to save energy. This is, however, counterbalanced by the need for an additional process step and the introduction of large quantities of a further substance into the process. For the distillation processes used to concentrate the aqueous raw product or to isolate the pentane-1,5-diamine from the extract, the use of a multiple column at a pressure of between 0.1 kPa and normal pressure is described in each case. No steps to enhance the energy efficiency in these distillation processes are mentioned.

A method is described in EP1602640A1 in which water from aqueous amine solutions, such as those produced in the hydrogenation of nitroaromatics, is separated in two distillation columns arranged in series. In the process, one of the columns is operated at 2 to 20 bar and the other at 0.1 to 10 bar. The condensation heat of the steam emerging from the column operated at higher pressure is used to evaporate water from the bottom of the column operated at lower pressure. The disclosure of this application is limited to the distillation of aromatic amines, such as those obtained from the hydrogenation of nitroaromatics. It is well known that these possess greater stability than aliphatic amines (see also EP1754698A2, paragraph [0004]). Depending on the distillation conditions, the distilled products still have a residual water content. By way of example, a residual water content of 3% is disclosed. No connection between the water content and the crystallisation behaviour of the amines is mentioned in this document.

Similar distillation arrangements for reducing energy consumption were described by O. Annakou and P. Mizsey in "Rigorous Comparative Study of Energy-Integrated Distillation" (Ind. Eng. Chem. Res. 1996, 35, 1877-1885). By referring to ternary mixtures of three alkanes in each case (butane to heptane), that article explains the possibilities for saving energy by adopting various concepts for thermal integration. Applying those concepts to remove water from compositions containing diamine is not mentioned anywhere.

Transport is made even more difficult for some important diamines by the fact that they have melting points between 5° C and 45° C. These are in particular the industrially relevant diamines pentane-1,5-diamine (PDA), hexane-1,6-diamine (HDA), 3-aminomethyl-3,5,5- trimethylcyclohexylamine (IPDA), bis(aminomethyl)norbornane (NBDA) and m-xylylene diamine (XDA). There is therefore a risk that during the transport of these diamines there may be phase transitions from solid to liquid or *vice versa,* which are undesirable for a number of reasons. Such phase transitions may be accompanied by the release of heat and therefore pose a risk to quality or even to transport safety. It is also a highly complex business to unload crystallised melt from a transport container or a transport ship. While it is possible to transport the diamines in heatable containers or ships, this is likewise undesirable.

EP0757034A1 describes a process for the production of toluylene diisocyanate in which first toluylene diamine is produced by the hydrogenation of dinitrotoluene. The processing by distillation of the aqueous raw toluylene diamine solution obtained is interrupted at a water content of 1 - 40 wt.-% based on the toluylene diamine and the mixture obtained in this way is transported to a different production location far away, where the processing by distillation is completed and, after that, the toluylene diamine is converted to toluylene diisocyanate with phosgene. It is disclosed that the raw toluylene diamine solutions result from the hydrogenation of dinitrotoluene with water contents of approx. 40%. With higher water contents, such as those which occur in the case of aliphatic diamines, especially aliphatic diamines from recycling processes or biotechnological production processes, there is a disadvantage, however, since drying by distillation entails considerable energy costs.

The problem of the present invention was to provide an improved method, offering economic and as far as possible also ecological benefits compared to the state of the art, in order to remove water at least partially from aliphatic diamines which are at first available as diluted aqueous solutions, and then to transport them over long distances and hence for lengthy periods of time and optionally to place them in temporary storage.

This problem has been solved by a method for the removal of water from and transport of at least one aliphatic diamine comprising the steps of:
a) providing a first composition including 60 to 98 wt.-% water and 2 to 40 wt.-% of the at least one aliphatic diamine with two ―NH₂ groups per molecule, each of them bound to a primary or secondary carbon atom,
b) removing water at least partially from said first composition by distillation at a first production location in a distillation apparatus comprising at least two distillation devices operating at different head pressures, thus generating a second composition comprising the diamine and ≤ 55 wt.-% water, wherein the vapours emerging from the distillation device operating at the higher head pressure are used to evaporate the liquid in the bottoms and/or the feed of a distillation device operating at a lower head pressure at least partially,
c) transporting said second composition during a span of time t from the first production location to a second production location, wherein the span of time t including times for optional temporary storage is at least 6 h.

In accordance with the invention, the expressions "comprising", "containing" or "including" preferably mean "consisting substantially of" and particularly preferably "consisting of".

Expressions such as "a *first partial stream and* a *second partial stream"* or "a *first distillation column and* a *second distillation column"* or "a *first part and* a *second part"* are always to be understood, unless specifically stated otherwise, as open wording, which does not rule out the presence of further (third, fourth, ...) partial streams, distillation columns or parts.

*Statements concerning the content of organic compounds* in the context of the present invention refer, unless stated otherwise, to values determined by gas chromatography. The skilled person is familiar with the quantitative evaluation of gas chromatograms, optionally using an internal standard. The skilled person is likewise familiar with any methods that might perhaps be needed to determine the *water content.* The methods known in the art can also be used in the context of the present invention. In case of doubt, the water content determined by Karl Fischer titration is decisive. The presence of amines can lead to trailing end points. In such cases, the figure determined by Karl Fischer titration after buffering with anhydrous benzoic acid is decisive. The skilled person is likewise familiar with the method using buffering with benzoic acid. For Karl Fischer titration in general, see Jander, Jahr, Massanalyse, 17th ed., de Gruyter, Berlin (2009), p. 279 to p. 282).

*"Distillation"* in the present case is understood to mean a thermal separation process used to recover evaporable substances, preferably liquids, from a composition. The separated vapours are subsequently precipitated, usually by condensation. The term encompasses in particular repeated evaporation and condensation using a column (distillation column) with a plurality of separating stages. Processes of this kind in which several distillation steps are arranged in series in a column should strictly speaking be called rectification, but will nevertheless likewise be referred to herein as distillation for the purpose of simplification. The advantage of these processes is the powerful separating effect and the possibility of operating the plant continuously. A *"distillation apparatus"* is accordingly an apparatus which is suitable for carrying out such a thermal separation process, i.e. for example sieve plate columns, packed columns, packed-bed columns, bubble cap tray columns or even single-stage evaporators such as falling film evaporators, thin-film evaporators, flash evaporators, multi-phase helical-coil evaporators, natural or forced circulation evaporators.

The expression *"back feed stream"* or *"back feed"* for short refers to that part of the condensed head product of a distillation column which is not removed as a product stream, but is returned to the distillation column or optionally to a different distillation column. The expression *"return ratio"* refers in the context of this description to the mass ratio of the back feed stream to the stream discharged. The return ratio is also referred to as the *"R*/*E ratio"* (back feed/withdrawal).

The relevant pressure of a distillation apparatus in the present case is the respective head pressure prevailing in the device, i.e. the pressure at which the vapours are present before they exit the distillation apparatus. This pressure will therefore also be referred to below as head pressure.

The individual process steps will be explained in more detail below.

In the first step a) of the method of the invention, a first composition is provided which includes 60 to 98 wt.-% water and 2 to 40 wt.-% of the at least one aliphatic diamine with two ―NH₂ groups per molecule, each of them bound to a primary or secondary carbon atom. Aliphatic diamines with two ―NH₂ groups in the context of the present invention mean diamines in which neither of the two NH₂ groups is bound to a carbon which is part of an aromatic ring system. They may therefore be linear aliphatic, branched aliphatic, cycloaliphatic or araliphatic diamines. Because of their generally high water solubility and high manufacturing costs compared to diamines with aromatically bound ―NH₂ groups, these are particularly suitable for the production processes mentioned, in which they are obtained mixed with water. They are therefore very particularly suitable for the method of the invention.

In the present case, "araliphatic" diamines means diamines which do contain an aromatic ring system, but in which the amino groups are not directly bound to that system, instead being located on two aliphatic side chains, each with at least one C-atom.

Isomeric mixtures of the same aliphatic diamine, for example in the case of bis(aminomethyl)norbornane, are considered like an aliphatic diamine for the present purposes. In this case, statements concerning relative contents for example refer to the total of all isomers.

The first composition preferably contains at least one aliphatic diamine selected from the group consisting of pentane-1,5-diamine (PDA), hexane-1,6-diamine (HDA), 3-aminomethyl-3,5,5-trimethylcyclohexylamine (IPDA), 2-methyl-1,5-diaminopentane (MPDA), the isomers of bis(aminomethyl)norbornane (NBDA), m-xylylene diamine (XDA), the isomers of methylcyclohexane diamine (H6-TDA), 1,3-bis(aminomethyl)cyclohexane (H6XDA) and mixtures of the aforementioned compounds. The first composition particularly preferably includes at least one aliphatic diamine selected from the group consisting of PDA, HDA, MPDA, IPDA, NBDA and XDA. Most particularly preferably, the first composition includes a linear aliphatic diamine selected from the group consisting of PDA and HDA.

In a preferred embodiment of the invention, the first composition is provided in the course of recycling polyurethanes, polythiourethanes, polyureas, polyisocyanurates and/or polyamides, such as PA64, PA66, PA69, PA610, PA612, PA54, PA56, PA59, PA510 or PA512, especially PA56 or PA66. Recycling is preferably by hydrolysis, i.e. breaking down the polymers in the presence of water to yield monomer building blocks. Hydrolysis may be performed in the presence of catalysts, preferably as alkaline hydrolysis. The polymers used for recycling may also contain allophanate and/or biuret groups. Compositions of this kind often contain large amounts of water, which may already be present during the reaction or may for example be introduced in a subsequent extraction step. Since water can be removed from the first composition in an energy-efficient manner with the method of the invention, it is now possible to use a larger amount of water in the recycling process if required.

In a further preferred embodiment of the invention, the first composition is provided by means of the biotechnological production of the diamine, for example by fermentation of suitable precursor compounds. In this case, the first composition is preferably a raw solution of the aliphatic diamine in water after any cellular components that may be present have been separated. Here too, the present invention allows greater dilution of the diamine during biotechnological production, since the removal of water using the method of the invention is particularly energy-efficient, so that the overall process remains economical.

In a preferred embodiment of the method of the invention, the first composition includes ≥3 wt.-% and ≤35 wt.-%, preferably ≥5% wt.-% and ≤25 wt.-% and particularly preferably ≥7 wt.-% and ≤22 wt.-% of the at least one aliphatic diamine with two ―NH₂ groups per molecule based on the total mass of the composition. In addition, the first composition usually also contains other substances apart from water which have a lower boiling point than the diamine. The content of these substances which have a lower boiling point than the amine is preferably ≤ 10 wt.-%, particularly preferably ≤ 5 wt.-% and most particularly preferably ≤ 3 wt.-%. At the same time, the content in technical processes is usually ≥ 0.1 wt.-%, preferably ≥ 0.5 wt.-%, unless low boilers have already been separated beforehand. Additionally, the first composition usually also contains other substances which have a higher boiling point than the amine. The proportion of these substances which have a higher boiling point than the amine in the first composition preferably totals ≥0.01 wt.-% and ≤10 wt.-%, particularly preferably ≥0.1 wt.-% and ≤5 wt.-%, and most particularly preferably ≥0.3 wt.-% and ≤3 wt.-%.

According to the invention, the water content of the first composition is ≥60 wt.-% and ≤98 wt.-%, preferably ≥62 wt.-% and ≤95 wt.-%, particularly preferably ≥65 wt.-% and ≤90 wt.-% and most particularly preferably ≥ 75 wt.-% and ≤88 wt.-% water based on the total mass of the first composition.

Substances with lower boiling points compared to the diamine that can occur in the first composition are, apart from water, for example ammonia, alkyl or alkenyl amines, alcohols, ethylene glycol, cyclic amines such as pyrrolidine, piperidine, azepane, imines, cylic imines such as tetrahydropyridine or tetrahydroazepine, or residues of solvents such as hydrocarbons or halogenated hydrocarbons for example. In addition, gases such as nitrogen or carbon dioxide may be present in the composition in dissolved or bound form.

Substances with higher boiling points compared to the diamine that can occur are for example dimers or oligomers of the diamine, which can form while cleaving off ammonia from the diamine. In particular in the preferred embodiment, in which, as a minimum, the at least one diamine in the first composition is produced by biotechnological means, the first composition may also contain carbohydrates which have not been completely converted in the fermentation process and salts that originate from the fermentation broth and are now still present in the first composition. If on the other hand the first composition originates from the chemical recycling of polyamides, such as PA66, preferably by acidic hydrolysis, it may also, in the case of the PA66 mentioned by way of example, contain adipic acid, salts thereof or fragments of PA66 as substances with higher boiling points. Statements concerning relative contents refer, unless stated otherwise, to the totality of the substances with higher boiling points compared to the amine.

The partial removal of water from the first composition provided in step a) is preferably performed continuously. It is performed in at least two distillation devices, with the vapours emerging from the distillation apparatus operated at a higher head pressure being at least partially condensed and the heat released in the process being used to heat the bottom of the distillation apparatus operated at a lower head pressure and/or to heat the feed stream into the distillation apparatus operated at a lower head pressure. The condensation temperature of the vapours naturally rises with the head pressure in the distillation apparatus concerned. It is desirable not to have an excessive increase in the heat transfer area required for the transfer of the condensation heat of the vapours to the feed or the bottom of the distillation apparatus operated at a lower head pressure. It is therefore expedient, in the case of each two distillation devices coupled by energy integration, for the ratio between the absolute head pressure in the distillation device operating at the higher head pressure and the absolute head pressure in the distillation device operating at the lower head pressure (higher head pressure in bar(a) : lower head pressure in bar(a)) to be at least 1.2 : 1, preferably at least 1.5 : 1 and particularly preferably at least 2 : 1. It is advantageous that in this way a logarithmic mean temperature difference of at least 5 K more preferably at least 7 K, particularly preferably 11 K, most particularly preferably between 14 K and 100 K is established at the heat exchanger used for thermal integration. If the temperature differences are too small, an excessively large heat transfer area is required and, depending on the operating principle of the heat exchanger used, this can interfere with the process, for example by causing the circulation in a natural circulation evaporator to come to a standstill because the temperature difference is too small.

It is preferable for the at least partial removal of water in step b) of the method of the invention to be performed in exactly two distillation devices with thermal integration, though it is in principle possible to use more than two distillation devices with different head pressures. Then it is preferable in each case to use the condensation heat from the stream of vapours of a distillation apparatus to heat the distillation apparatus with the next-lower head pressure. It should be noted in this context that the investment costs for the devices increase, while the additional energy savings decrease with each further distillation device. As a rule, it is therefore ideal for economic reasons to perform the at least partial removal of water in exactly two distillation devices. Because of the higher separating performance of distillation columns compared to single-stage evaporators, it is particularly preferable to carry out the at least partial removal of water in exactly two distillation columns, i.e. for example sieve plate columns, packed columns, packed-bed columns or bubble cap tray columns.

In the following paragraphs, the invention will be explained in more detail with reference to the most preferred embodiment with *exactly two distillation columns.* Starting from this explanation, it is readily possible for the skilled person to transfer the invention to other embodiments.

It is possible to use the heat exchange from the vapours of the first distillation column, i.e. the column operated at a higher head pressure, to heat the second distillation column, i.e. the column operated at a lower head pressure, indirectly by first using the condensation heat to generate water vapour and then to use that to heat the second distillation column. In this case, at least two evaporator-condensers and a greater pressure difference between the two columns are needed. It is therefore preferable to carry out the heat exchange directly (i.e. without an additional heat transfer medium).

The removal of water in step b) can be performed in a distillation apparatus in which the columns are arranged in parallel. In this embodiment, the composition provided in step a) is divided into two or more partial streams which then serve as feed streams for the distillation columns, the number of partial streams preferably corresponding to the number of distillation columns switched in parallel, and each column receiving at least one, preferably exactly one, partial stream as feed. It is clear to the skilled person that distillation devices other than distillation columns can likewise be arranged in parallel.

Alternatively, the removal of water in step b) can be performed in a distillation apparatus in which the columns are arranged in series. In this embodiment, the composition provided in step a) is delivered as feed to one of the two columns, and the bottom product of that column forms the feed for the second column. It is possible in this case to operate the former column at a higher head pressure than the latter column (forward match) or *vice versa* to operate the latter column at a higher head pressure than the former (reverse match). The forward match is preferred because in this case the bottom product from the first column can flow into the second column without even using a pump if the pressure difference is sufficiently high. It is clear to the skilled person that the distillation devices can likewise be arranged in series, preferably in a forward match.

Irrespective of the arrangement of the columns, water, possibly in the form of azeotropes with other compounds, is separated in accordance with the invention and recovered at the head of the column as steam (vapours) in all the columns. The vapours from the column with the higher head pressure are preferably condensed in an evaporator-condenser, which serves either as a circulation evaporator at the bottom of the column with the lower head pressure or as a pre-evaporator which at least partially evaporates the feed to the column with the lower head pressure. In the process, the vapours from the column with the higher head pressure are condensed. The condensate can be supplied, optionally after further intermediate steps such as phase drying, at least partially as back feed to one or more of the columns. The remaining part or the entire condensate can be processed, used, recycled or disposed of in some other way. Portions of the vapours which are not condensible under the conditions prevailing in the evaporator-condenser can be fed to the condenser of the column operated at a lower head pressure, which in the case of more than two columns is preferably the condenser of the column operated at the lowest head pressure, and further condensed there. This prevents inert substances from accumulating in the evaporator-condenser and impairing its thermal transfer performance.

When the columns are arranged in series, the second composition containing the diamine and ≤55 wt.-% water is obtained as a bottom product of the downstream column. The water content is dependent on the operating conditions such as the ratio of the inlet mass flow to bottoms mass flow, the input of heating energy and the return ratio. Depending on the composition of the substances and the pressure, an evaporation temperature sets in at the bottom (bottom temperature) which can also be used to monitor the process and influence the parameters in such a way that the desired water content in the second composition is achieved.

When the columns are arranged in parallel, at each of the at least two columns part of the second composition containing the diamine and ≤55 wt.-% water is formed at the bottom of the respective column. The compositions of the two (or more) partial streams of the composition may differ because of different process conditions in the columns. After removal from the columns, it is expedient to join the partial streams and to subject them to further process steps together. It is of course equally possible deliberately to produce two or, depending on the number of columns, even more different qualities of the second composition and process them separately.

If the intention is to achieve low water contents in the second composition, it is preferable to arrange the distillation columns in series in a forward match, which means that the first composition is delivered as feed to the first column and the latter is operated at a higher pressure than the second column. The second column then receives the bottom product of the first column as feed, and the vapours of the first column are used to heat the feed and/or the bottoms of the second column and preferably partially to evaporate them. In this arrangement, a greater proportion of water remains in the bottom product of the first column, which lowers the evaporation temperature in the bottom of that column and thus reduces the temperature difference inside the column, i.e. between the vapours and the bottom of the first column. This effect can be exploited to operate the column at a higher pressure and at the same time to keep down the thermal load on the amine present in the bottom. In order to achieve the low amount of water in the second composition and hence in the bottom of the second column, there has to be a high temperature there. The effect described concerning the residual water in the bottom of the first column therefore makes it possible to increase the pressure in the first column and thus to raise the temperature of the first column - while subjecting the amine to only a small thermal load - so far that the condensation heat of the vapours can be utilised in accordance with the invention. The objectives of a high vapour temperature and a low bottom temperature in the first column, which are actually conflicting, can be better reconciled in this way.

For diamines and transport or storage conditions where phase transitions do not present a problem, the water content of the second composition is preferably ≤ 10 wt.-%, particularly preferably ≤ 1 wt.-%, very particularly preferably ≤ 0.1 wt.-% and most preferably ≤ 0.05 wt.-% based on the entire second composition. On the other hand, extremely dry amines can only be produced with considerable effort, so that the water content is preferably ≥ 0.0001 wt.-%, particularly preferably ≥ 0.001 wt.-% and most particularly preferably ≥ 0.01 wt.-%.

Some aliphatic diamines have melting points between 5° C and 45° C. These are in particular the industrially relevant diamines pentane-1,5-diamine (PDA), hexane-1,6-diamine (HDA), 3-aminomethyl-3,5,5- trimethylcyclohexylamine (IPDA), bis(aminomethyl)norbornane (NBDA) and m-xylylene diamine (XDA). The highest melting points among these diamines are found in HDA at 40.5° C, NBDA at approx. 16° C and XDA at 14° C.

For these diamines in particular, it may make sense to set the water content in the second composition at somewhat higher levels in order to keep the risk of phase transitions during transport and/or temporary storage to a minimum. At the same time, the water content should not be adjusted to too high a level in order not to render the transport inefficient. In a further preferred embodiment, the water content of the second composition is therefore ≥1 and ≤35 wt.-%, preferably ≥2 and ≤27 wt.-% and particularly preferably ≥5 and ≤20 wt.-% based on the entire second composition.

Since the second composition in the method of the invention is formed as a bottom product of distilllation, it usually contains small amounts of dimerisation products of the general formula (I) or (II):

H₂N-R¹-NH-R¹-NH₂ (I)

H₂N-R¹-N=R²-NH₂ (II)

where R¹ in formula (I) or (II) is a linear aliphatic, branched aliphatic, cycloaliphatic or araliphatic hydrocarbon residue which is derived from the respective diamine by abstraction of the two NH₂ groups, and R² in formula (II) is a linear aliphatic, branched aliphatic, cycloaliphatic or araliphatic hydrocarbon residue which is derived from the respective diamine by abstraction of the two NH₂ groups and in addition to that of a hydrogen atom from a carbon atom which also carried one of the two NH₂ groups groups. R¹ in formula (I) or (II) preferably stands for a hydrocarbon residue which is derived from pentane-1,5-diamine (PDA), hexane-1,6-diamine (HDA), 3-aminomethyl-3,5,5- trimethylcyclohexylamine (IPDA), 2-methyl-1,5-diaminopentane (MPDA), the isomers of bis(aminomethyl)norbornane (NBDA, m-xylylene diamine (XDA), the isomers of methylcyclohexane diamine (H6-TDA) or 1,3-bis(aminomethyl)cyclohexane (H6XDA) by abstraction of the two NH₂ groups, and R² in formula (II) stands for a hydrocarbon residue which is derived from pentane-1,5-diamine (PDA), hexane-1,6-diamine (HDA), 3-aminomethyl-3,5,5-trimethylcyclohexylamine (IPDA), 2-methyl-1,5-diaminopentane (MPDA), the isomers of bis(aminomethyl)norbornane (NBDA), m-xylylene diamine (XDA), the isomers of methylcyclohexane diamine (H6-TDA) or 1,3-bis(aminomethyl)cyclohexane (H6XDA) by abstraction of the two NH₂ groups and additionally of an H-atom from a carbon atom to which one of the two NH₂ groups was bound, and wherein R¹ and R² are preferably based on the same diamine. Particularly preferably, R¹ in formula (I) or (II) stands for -CH₂CH₂CH₂CH₂CH₂CH₂- and R² in formula (II) for =CHCH₂CH₂CH₂CH₂CH₂- or R¹ in formula (I) or (II) for - CH₂CH₂CH₂CH₂CH₂- and R² in formula (II) for =CHCH₂CH₂CH₂CH₂-. Dimers of this kind are used for example in order to incorporate functionalities into diamine-based polymers and to modify their properties.

A further subject-matter of the invention is therefore second compositions including, based on the total mass of the second composition, 0.01 wt.-% to 55 wt.-%, preferably 1 wt.-% to 35 wt.-% and particularly preferably 2 wt.-% to 27 wt.-% water, 45 wt.-% to 99 wt.-%, preferably 65 wt.-% to 99 wt.-% of an aliphatic diamine and in total 0.0005 wt.-% to 5 wt.-%, preferably 0.001 wt.-% to 3 wt.-% and particularly preferably 0.01 wt.-% to 2 wt.-% of dimeric compounds according to formula (I) and formula (II).

In the distillation columns, a falling liquid phase and a rising steam phase are brought together in counterflow. In the process, heat and mass transfer occurs, which causes the substances to separate. The columns preferably contain separation supporting structures to enlarge the mass transfer area. These structures may for example be different kinds of mass transfer trays (bubble cap trays, sieve trays, valve trays, etc.), packings with a random arrangement of packing material or structured packings, the latter usually involving a low pressure loss and therefore being preferable. The separation performance of a distillation column is characterised by the number of its theoretical trays, i.e. the number of mass transfer trays on which there is an equilibrium between the liquid running down and the steam rising. Since no equilibrium is achieved on a tray in reality, and there are no real trays in packed columns, the number of trays physically present is usually different from the number of theoretical trays, but the concept of theoretical trays can still be transferred to all separation supporting structures, so that in the present case it is convenient to speak in general terms of theoretical stages (or synonymously of theoretical trays), where a theoretical stage causes enrichment in accordance with the thermodynamic equilibrium.

The columns used to carry out the method of the invention preferably have 2 to 50 theoretical stages. The column with the lower pressure is preferably operated at a bottom temperature of 50 to 180° C, particularly preferably at a bottom temperature of 80 to 160° C. The pressure at the head of the column is preferably 0.05 to 10.0 bar(a), particularly preferably 0.1 to 5.0 bar (a) and most particularly preferably 0.12 to 2.0 bar(a). With a suitable choice of the absolute pressure at the head of the column with the lower head pressure, the vapours of that column can also be used for example to evaporate solvents, to heat product streams or to generate water vapour.

The column with the higher pressure is preferably operated at a bottom temperature of 95 to 240° C, particularly preferably 120 to 180° C. The absolute pressure at the head of the column is preferably 0.8 to 20.0 bar(a), particularly preferably 1.5 to 10.0 bar(a), most particularly preferably 2.5 to 7.0 bar(a). When choosing the head pressure, it is of course the case that it is necessary to remember the general condition mentioned earlier, namely that the vapours from this column should condense at a temperature which is sufficiently far above the evaporation temperature in the bottom of the column operated at a lower pressure.

At the bottom of the distillation column operated with a higher head pressure there is at least one device for delivering heat, with which part of the bottoms is evaporated. This may be a tube bundle in the interior of the column bottom, which is for example heated by means of water vapour that condenses inside the tube and transfers the condensation heat released in the process via the tube walls to the content of the column bottom. It is preferably an external circulation evaporator, i.e. for example a tube bundle heat exchanger, into which a bottom stream is either drawn in automatically (natural circulation evaporator) or is conveyed in by means of a pump (forced circulation evaporator) and which is heated for example by means of water vapour. The device for delivering heat causes part of the bottoms to be evaporated and then to rise in the column as steam, in the course of which it comes into contact with the liquid running down. This ultimately results in the partial removal of water from the first composition.

The column operated at a lower head pressure is likewise in fluid connection with at least one device for delivering heat. At least one of these devices is heated by the vapours exiting the column operated at a higher head pressure. This heating can take place in the feed of the column operated at a lower head pressure and/or at the bottom of that column. At the bottom of the second column there is optionally a further device for delivering heat, with which part of the bottoms can be evaporated. This may be a tube bundle in the interior of the column bottom, which is for example heated by means of water vapour, or an optional further circulation evaporator at the bottom of the column. In standard operation, such additional heating by means of water vapour is not vitally necessary, but it is very useful for example for commissioning processes or in order to compensate for process fluctuations.

The second composition, which is formed in the course of distillation, is transported, preferably in liquid form, from the first production location to a second production location over a span of time t. The transport is preferably carried out in vessels selected from the group consisting of transport containers, tank waggons or tankers. Transport in tankers is particularly preferable. The span of time t includes not only the actual transport, i.e. the process of shipping the second composition from one production location to the other production location, but also any temporary storage in tanks that may be necessary after the removal of water at the first production location and before phosgenation or polycondensation, and any further purification that may be necessary, at the second production location. It thus corresponds to the span of time from when the second composition is filled into a storage tank or transport container after it has been removed from the distillation apparatus until the time when the second composition is fed from a storage tank or transport container into a process for further use to be made of the second composition at the second production location. According to the invention, the span of time t is at least 6 h. The longer the span of time, the greater the benefits that can be reaped from the method of the invention. This is why in a further preferred embodiment the span of time t is at least 24 h, preferably at least 168 h and particularly preferably at least 336 h.

The second composition, from which at least part of the water has been removed in this way and which has been transported to the second production location, is particularly suitable for the production of aliphatic diisocyanates, polyamides or diamine-dicarboxylic acid salts. It is therefore also preferable for the method of the invention to comprise the following steps after step c):
d) optionally further purifying the second composition at the second production location, thus generating a third composition,
   and either
e) phosgenating the second and/or the optionally obtained third composition at the second production location so that at least one aliphatic diisocyanate is generated
   or
f) polycondensing the second and/or the optionally obtained third composition at the second production location so that a polyamide is generated
   or
g) converting the second and/or the optionally obtained third composition with a dicarboxylic acid to the diamine-dicarboxylic acid salt.

A further subject-matter of the invention is therefore a method for producing a polyamide or an aliphatic diisocyanate comprising the steps of:
a) providing a first composition including 60 to 98 wt.-% water and 2 to 40 wt.-% of the at least one aliphatic diamine with two ―NH₂ groups per molecule, each of them bound to a primary or secondary carbon atom,
b) removing water at least partially from said first composition by distillation at a first production location in a distillation apparatus comprising at least two distillation columns operating at different head pressures, thus generating a second composition comprising the diamine and ≤ 55 wt.-% water, wherein the vapours emerging from the distillation column operating at the higher head pressure are used to evaporate the liquid in the bottoms and/or the feed of a distillation column operating at the lower head pressure at least partially,
c) transporting said second composition during a span of time t from the first production location to a second production location, wherein the span of time t including times for optional temporary storage is at least 6 h,
d) optionally further purifying the second composition at the second production location, thus generating a third composition,
   and either
e) phosgenating the second and/or the optionally obtained third composition at the second production location so that at least one aliphatic diisocyanate is generated
   or
f) polycondensing the second and/or the optionally obtained third composition at the second production location so that a polyamide is generated
   or
g) converting the second and/or the optionally obtained third composition with a dicarboxylic acid to the diamine-dicarboxylic acid salt.

After the transport to the second production location and any temporary storage at the second production location that may be required, the second composition may if necessary be subjected to further purification so that a third composition is generated. This is preferably done by distillation or a combination of distillation and extraction steps. The skilled person is sufficiently familiar with processes for doing this.

The purification of diamines by distillation is usually performed in, for example, a distillation sequence involving several steps. First of all, water and certain low boilers, especially those which form an azeotrope with water, are separated in a dewatering column over the head. Depending on the configuration of the water removal process in step b) of the method of the invention, this step might be dispensed with if it has been carried out completely at the first production location. Even if any residual amounts of water remaining do not disturb the further conversion, for example because this takes place in the aqueous medium anyway, this first step may perhaps be dispensed with in purification by distillation. After that, high and low boilers are separated, with the steps performed either in individual distillation columns or combined in a dividing wall column. If two separate distillation columns are used, it is preferable first to remove the high boilers from a distillation column as a bottom product and only then to withdraw the further low boilers over the head of a further distillation column. Finally, it is preferable to perform a fine distillation step yielding the third composition.

The second composition and/or the optionally obtained third composition can be converted with phosgene in step e) of the method of the invention to yield at least one aliphatic diisocyanate.

Alternatively, it can be converted in a polycondensation reaction with a dicarboxylic acid or a dicarboxylic acid derivative in step f) of the method of the invention so that a polyamide is generated. Since the second or third composition is substantially the at least one aliphatic diamine, the expression "aliphatic diamine" will be used instead of the expressions "second composition" and "third composition" in the following description of the phosgenation reaction and the polycondensation in order to render the text more easily readable.

The aliphatic diamine can be converted into a polyamide in step f) in a polycondensation reaction with a dicarboxylic acid and/or a dicarboxylic acid derivative selected from the group consisting of dicarboxylic acid diesters, dicarboxylic acid dichlorides and dicarboxylic acid anhydrides. It is preferable for the polycondensation to be performed with a dicarboxylic acid with elimination of water. In this embodiment, the aliphatic diamine is heated in an aqueous solution together with a dicarboxylic acid and is thus converted into the polyamide in a polycondensation reaction. Optional intermediate steps may be performed, such as the production and isolation of the salt from the aliphatic diamine and the dicarboxylic acid for example. The conversion is preferably performed under pressure, with the pressure being controlled by deliberately releasing surplus water vapour. In order to build up high molecular weights, the pressure is reduced in the further course of the reaction and the water is preferably removed from the reaction mixture completely.

A further alternative is to convert the aliphatic diamine with a dicarboxylic acid to the diamine-dicarboxylic acid salt in step g). The conversion is preferably performed in an aqueous solution, with the equivalence of the components preferably being adjusted via the pH. If it is to be expected, for example, that diamine losses will occur in a later polymerisation of the AH salt, a slight stoichiometric excess of 0.5% to 5%, preferably from 1% to 3% of the diamine compared to the carboxylic acid can be used in the production of the diamine-dicarboxylic acid salt, so that there is a pH of more than 7.0. The salt can be crystallised by evaporating water and/or cooling the hot solution. If necessary, it can be purified by recrystallisation, for example from methanol.

The phosgenation of the aliphatic diamine in step e) may for example be performed in the gaseous phase. The skilled person is sufficiently familiar with methods for the gas phase phosgenation of aliphatic diamines from the state of the art. Phosgenation is performed at temperatures ranging from 200 to 600° C with an excess of phosgene, optionally in the presence of an inert gas or vapours of an inert solvent.

After conversion, which is performed in a preferably cylindrical reaction space, the diisocyanate formed is removed from the reaction mixture, preferably by selective condensation in an inert solvent such as chlorobenzene or dichlorobenzene, and is then processed into pure diisocyanate in a multiple distillation process.

In a further embodiment, the phosgenation of the aliphatic amine takes place in the liquid phase. The reaction can then be performed in various ways. Either the aliphatic amine is reacted directly with an excess of phosgene in an inert liquid medium, preferably in a two-stage process known as cold-hot phosgenation (base phosgenation) or it is first transformed into the corresponding salt by conversion with hydrogen chloride gas or carbon dioxide in an inert liquid medium and then converted with an excess of phosgene similar to the hot phosgenation step in base phosgenation (hydrochloride or carbaminate phosgenation). A suitable liquid medium for all phosgenations is in particular chlorobenzene and/or dichlorobenzene. The skilled person is also familiar with these processes for phosgenation in the liquid phase.

Both in base phosgenation and in amine-hydrochloride or carbaminate phosgenation, the remaining phosgene and hydrogen chloride gas is preferably blown off with an inert gas, preferably nitrogen, after the reaction is completed. If needed, filtration may be performed in order to remove any solids that might be present, such as unreacted amine-hydrochlorides. The diisocyanate formed is then processed by multiple distillation to the pure diisocyanate.

### Examples

The invention will now be explained with reference to examples. Examples 1 to 4 are simulations based on the thermodynamic data of the compounds, which were performed using Aspen Plus^{®}. As the model system for the first composition to simplify matters, a mixture consisting of 80% water and 20% aliphatic diamine was assumed, on the basis of which the energy savings for different variants of the water removal process are demonstrated. The removal of water was performed in a distillation system of two columns with thermal integration in the form of a forward match. Both distillation columns were assumed to have 30 stages and to have a feed divider at the head.

The first composition was heated in a preheating step from 40° C to 100° C and fed into the 20th stage of the first column, where part of the feed was distilled off over the head. The distillate was condensed and partially removed as a distillate stream, and partially fed to the first column as back feed, so that the distillate was largely free of amine. The bottom product was for its part introduced into the 20th stage of the second column. The heating energy for that column was in each case recovered from the steam condensation of the first column, and the return ratio was adjusted such that the desired residual water content was established in the bottom of the column. As a measure of the energy saving by the forward match, it was determined what share of the heating energy is contributed by the steam condensation of the first column to the total heating energy, i.e. to the total heating energy requirements of the preheater, the first column and the second column. That share of the heating energy can therefore be saved compared to a simple process for removing water by distillation in a column without thermal integration as is known in the art.

Whereas the water was removed almost completely in Examples 1 and 3, a residual water content of 10% in the second composition was allowed in Examples 2 and 4. In Example 2, the pressure level in the second column, i.e. the column with the lower head pressure, was set at 1.5 bar(a), which meant that the condensation heat of the vapours from this column were just high enough to evaporate and overheat water at atmospheric pressure for example. In Example 4, on the other hand, the pressure level in the two columns was reduced so far that the bottom temperature in the first column was 139° C. In this way, in contrast to the other examples, it was possible to heat it with water vapour at 6 bar(a), whereas steam on a higher pressure level was needed in the other examples.

**Table 1: Summary of Examples 1 to 4**

| Example | 1 | 2 | 3 | 4 |
|---|---|---|---|---|
| Diamine used | PDA | PDA | HDA | HDA |
| Head pressure first column [bar(a)] | 5.5 | 5.0 | 5.0 | 3.3 |
| Distillate-to-feed ratio 1st column [m/m] | 0.45 | 0.45 | 0.45 | 0.45 |
| Return ratio first column | 0.2 | 0.2 | 0.2 | 0.2 |
| Bottom temperature first column | 158 | 155 | 155 | 139 |
| Condensation temperature first column [°C] | 156 | 152 | 152 | 137 |
| Head pressure second column [bar(a)] | 0.25 | 1.5 | 0.15 | 0.75 |
| Return ratio second column | 0.145 | 0.163 | 0.144 | 0.168 |
| Bottom temperature second column [°C] | 134 | 138 | 138 | 116 |
| Condensation temperature second column [°C] | 65 | 111 | 54 | 92 |
| Residual water content in the second composition [wt.-%] | 0.05 | 10 | 0.05 | 10 |
| Share of heating energy from steam condensation | 40% | 40% | 40% | 42% |

### Example 5:

By way of example, the setting points of various mixtures consisting of hexane-1,6-diamine and water or pentane-1,5-diamine and water depending on the water content were determined. The results are summed up in the following Table. The results, taking the transport and storage conditions into account, give the skilled person an indication of the residual water content that must be present in the second composition in order reliably to rule out phase transitions from liquid to solid during the transport or temporary storage of the second composition.

**Table 2: Survey of the solidification temperatures of hexane-1,6-diamine/water mixtures**

| Water content [wt.-%] | 0.0 | 1.0 | 3.0 | 5.0 | 10.0 | 15.0 | 20.0 | 27.0 | 33.0 | 38.0 |
|---|---|---|---|---|---|---|---|---|---|---|
| Solidification temperature hexane-1,6-diamine/water [°C] | 40.5 | 39.4 | 37.2 | 35.0 | 29.5 | 23.8 | 17.8 | 9.5 | 2.5 | -3.0 |
| Solidification temperature pentane-1,5-diamine/water [°C] | 15.1 | 14.0 | 11.7 | 9.7 | 4.6 | -2.6 | -12.2 | -29.2 | -38.2 | -43.9 |

## Claims

1. Method for the removal of water from and transport of at least one aliphatic diamine comprising the steps of:
a) providing a first composition including 60 to 98 wt.-% water and 2 to 40 wt.-% of the at least one aliphatic diamine with two ―NH₂ groups per molecule, each of them bound to a primary or secondary carbon atom,
b) removing water at least partially from said first composition by distillation at a first production location in a distillation apparatus comprising at least two distillation devices operating at different head pressures, thus generating a second composition comprising the diamine and ≤ 55 wt.-% water, wherein the vapours emerging from the distillation device operating at the higher head pressure are used to evaporate the liquid in the bottoms and/or the feed of a distillation device operating at the lower head pressure at least partially,
c) transporting said second composition during a span of time t from the first production location to a second production location, wherein the span of time t including times for optional temporary storage is at least 6 h.

2. The method according to claim 1, **characterised in that** the at least one aliphatic diamine is selected from the group consisting of pentane-1,5-diamine, hexane-1,6-diamine, 3-aminomethyl-3,5,5-trimethylcyclohexylamine, the isomers of bis(aminomethyl)norbornane, m-xylylene diamine, the isomers of methylcyclohexane diamine, 1,3-bis(aminomethyl)cyclohexane and mixtures of the aforementioned compounds.

3. The method according to claim 1 to 2, **characterised in that** the first composition provided in step a) is obtained by recycling polyurethanes, polythiourethanes, polyureas, polyisocyanurates and/or polyamides.

4. The method according to claim 1 to 2, **characterised in that** the first composition provided in step a) is obtained by biotechnological production of the diamine, for example by fermentation of suitable precursor compounds.

5. The method according to any one of claims 1 to 4, **characterised in that** the first composition includes ≥3 wt.-% and ≤35 wt.-%, preferably ≥5% wt.-% and ≤25 wt.-% and particularly preferably ≥ 7 wt.-% and ≤22 wt.-% of the at least one aliphatic diamine.

6. The method according to any one of claims 1 to 5, **characterised in that** the water content of the first composition is ≥62 wt.-% and ≤95 wt.-%, preferably ≥65 wt.-% and ≤90 wt.-% and particularly preferably ≥75 and ≤88 wt.-% based on the total mass of the first composition.

7. The method according to any one of claims 1 to 6, **characterised in that** for each two distillation devices coupled by energy integration the ratio between the absolute head pressure in the distillation device operating at a higher head pressure and the absolute head pressure in the distillation device operating at a lower head pressure is at least 1.2 : 1, preferably at least 1.5 : 1 and particularly preferred at least 2 : 1.

8. The method according to any one of claims 1 to 7, **characterised in that** the removal of water in step b) of the method of the invention is performed in exactly two distillation devices with thermal integration, preferably exactly two distillation columns with thermal integration.

9. The method according to any one of claims 1 to 8, **characterised in that** the removal of water in step b) is performed in a distillation apparatus in which the distillation devices are arranged in parallel.

10. The method according to any one of claims 1 to 8, **characterised in that** the removal of water in step b) is performed in a distillation apparatus in which the distillation devices are arranged in series, preferably in a forward match.

11. The method according to any one of claims 1 to 10, **characterised in that** the water content of the second composition is ≤ 10 wt.-%, preferably ≤ 1 wt.-%, particularly preferably ≤ 0.1 wt.-% and very particularly preferably ≤ 0.05 wt.-% based on the entire second composition.

12. The method according to any one of claims 1 to 10, **characterised in that** the water content of the second composition is ≥1 and ≤35 wt.-%, preferably ≥2 and ≤27 wt.-% and particularly preferably ≥5 and ≤20 wt.-% based on the entire second composition.

13. The method according to any one of claims 1 to 12, **characterised in that** the transport is carried out in vessels selected from the group consisting of transport containers, tank waggons or tankers.

14. The method according to any one of claims 1 to 13, **characterised in that** the span of time is t at least 24 h, preferably at least 168 h and particularly preferably at least 336 h.

15. Method for producing a polyamide or an aliphatic diisocyanate comprising the steps of:
a) providing a first composition including 60 to 98 wt.-% water and 2 to 40 wt.-% of the at least one aliphatic diamine with two ―NH₂ groups per molecule, each of them bound to a primary or secondary carbon atom,
b) removing water at least partially from said first composition by distillation at a first production location in a distillation apparatus comprising at least two distillation devices operating at different head pressures, thus generating a second composition comprising the diamine and ≤ 55 wt.-% water, wherein the vapours emerging from the distillation device operating at the higher head pressure are used to evaporate the liquid in the bottoms and/or the feed of a distillation device operating at the lower head pressure at least partially,
c) transporting said second composition during a span of time t from the first production location to a second production location, wherein the span of time t including times for optional temporary storage is at least 6 h,
d) optionally further purifying the second composition at the second production location, thus generating a third composition,
and either
e) phosgenating the second and/or the optionally obtained third composition at the second production location so that at least one aliphatic diisocyanate is generated
or
f) polycondensing the second and/or the optionally obtained third composition at the second production location so that a polyamide is generated
or
g) converting the second and/or the optionally obtained third composition with a dicarboxylic acid to the diamine-dicarboxylic acid salt.

16. A second composition, comprising based on the total mass of the second composition 0.01 wt.-% to 55 wt.-% water, 45 wt.-% to 99 wt.-% of an aliphatic diamine and in total 0.0005 wt.-% to 5 wt.-% of dimeric compounds according to formula (I) and formula (II):
H₂N-R-NH-R-NH₂ (I)
H₂N-R-N=R-NH₂ (II)
where R¹ in formula (I) or (II) is a linear aliphatic, branched aliphatic, cycloaliphatic or araliphatic hydrocarbon residue which is derived from the respective diamine by abstraction of the two NH₂ groups and R² in formula (II) is a linear aliphatic, branched aliphatic, cycloaliphatic or araliphatic hydrocarbon residue which is derived from the respective diamine by abstraction of the two NH₂ groups and in addition to that of a hydrogen atom from a carbon atom which also carried one of the two NH₂ groups.
